# EUROPÄISCHE PATENTSCHRIFT

(11) **EP 0 874 870 B1**
(45) Veröffentlichungstag und Bekanntmachung des Hinweises auf die Patenterteilung: **26.03.2003**
(21) Anmeldenummer: 97900971.9
(22) Anmeldetag: 09.01.1997
(51) Int. Cl.: C08G 65/32, C07C 67/03

(54) **VERFAHREN ZUR HERSTELLUNG VON POLYGLYCOL(METH)ACRYLATEN**
PROCESS FOR THE PREPARATION OF POLYGLYCOL(METH)ACRYLATES
PROCEDE DE PRODUCTION DE POLYGLYCOL(METH)ACRYLATES

(30) Priorität: 20.01.1996 DE 19602035
(43) Veröffentlichungstag der Anmeldung: 04.11.1998
(73) Patentinhaber: Röhm GmbH & Co. KG, 64293 Darmstadt (DE)
(72) Erfinder: KNEBEL, Joachim, D-64293 Darmstadt (DE); SPALT, Werner, D-64331 Weiterstadt (DE)
(86) Internationale Anmeldenummer: EP9700056
(87) Internationale Veröffentlichungsnummer: WO97026293

(56) Entgegenhaltungen:
- DE-A- 4 019 788
- US-A- 4 672 105
- US-A- 5 362 904
- DATABASE WPI Section Ch, Week 9542 Derwent Publications Ltd., London, GB; Class A25, AN 95-325506 XP002029813 & JP 07 224 004 A (YOKKAISHI GOSEI KK) , 22.August 1995

## Beschreibung

### Gebiet der Erfindung

Die Erfindung betrifft ein Verfahren zur Herstellung von Alkoxypolyglycol(meth)acrylaten durch basisch katalysierte Umesterung, wobei als Katalysatoren ein Gemisch aus Lithiumchlorid und Calciumhydroxid oder Calciumhydroxid allein verwendet wird.

### Stand der Technik

Ester der (Meth)acrylsäure mit Polyethylenglykolen bzw. den Monoetherderivaten derselben sind technisch insbesondere als dispergierwirksame Monomere von nicht geringem Interesse. Umso wichtiger ist es ein möglichst günstiges Herstellungsverfahren zur Verfügung zu haben.

Die Umesterung der (Meth)acrylsäurealkylester von niederen Alkanolen insbesondere der Methyl- und Ethylester mit höheren bzw. mit substituierten Alkoholen unter Katalyse durch verschiedenartige Katalysatoren stellt eine relative elegante Methode zur Herstellung von höheren bzw. komplexeren Estern der (Meth)acrylsäure dar.

Zu den wegen ihrer vielfältigen Einsatzmöglichkeiten interessanten (Meth)acrylsäureestem gehören diejenigen mit Alkoxypolyglykolen.

Die Umesterungsreaktion der genannten, niederen (Meth)acrylsäureester mit Alkoxypolyglykolen verläuft beispielsweise unter Katalyse durch die einschlägig verwendeten Ortho-Titansäureester (vgl. GB 960 005; 962 928; DE-A 4 121 811) zwar einigermaßen befriedigend, jedoch gestaltet sich die Aufarbeitung der Umesterungsansätze ungewöhnlich schwierig. Zwar erhält man beispielsweise unter Katalyse mit Tetraisopropyltitanat ein Produkt in guter Ausbeute und mit geringer Eigenfarbe, bei der unvermeidlichen Überführung des Titanats in das unlösliche, hydratisierte Titandioxid durch Wasserzusatz erhält man aber ein extrem schlecht filtrierbares Hydrolyseprodukt. Der Filtrationsprozeß nimmt bei Durchführung des Verfahrens im Produktionsmaßstab viel zu lange Zeit in Anspruch, so daß sich die Aufarbeitung über Tage erstrecken kann.

Als Alternative zur Titanatkatalyse kommt z. B. die Katalyse durch Alkalialkoxide (CH-PS 239 750) oder mit einer bestimmten Lithiumverbindungen insbesondere Lithiumhydroxid vorzugsweise in Kombination mit Calciumoxid enthaltendem Katalysator in Frage (vgl. US-A 4 672 105; US-A 4 745 213; DE-A 2 744 641; GB-C 1 094 998).

### Aufgabe und Lösung

Angesichts der vorstehend dargestellten Erfahrungen mit den im allgemeinen brauchbaren Katalysatoren auf Titanat-Basis bestand ein besonderes Bedürfnis, ein effektives und kostengünstiges Umesterungsverfahren zur Herstellung von (Meth)acrylsäureestem von gegebenenfalls monoveretherten Polyethylenglykolen zur Verfügung zu haben.

Entsprechende Versuche mit dem als Alternative zur Titanatkatalyse sehr erfolgreich eingesetzten Katalysator Lithiumhydroxid bzw. den Katalysatorensystemen aus Lithiumhydroxid und Calciumoxid bzw. Lithiumchlorid und Calciumoxid verliefen indessen enttäuschend. Setzt man beispielsweise Methylmethacrylat mit Methoxypolyethylenglykol unter Katalyse mit den genannten Umesterungskatalysatoren unter den mit sonstigen Alkoholen als Edukten bewährten Bedingungen um, so erhält man gelb bis rotbraun gefärbte Produkte, die in dieser Form unverkäuflich sind. (Für das Katalysatorensystem Lithiumhydroxid/Calciumoxid waren z. B. in der US-A 4 672 105 bei der Umsetzung von Polyolen mit die besten Ausbeuten berichtet worden).
Damit schien auch dieser Typ von Umesterungskatalysatoren zur Lösung der vorliegenden Aufgabe ungeeignet zu sein.
Es wurde nun gefunden, daß die vorliegende Aufgabe, ein in jeder Hinsicht befriedigendes Umesterungsverfahren zur Herstellung von (Meth)acrylsäureestern von Polyethylenglykolen und Polypropylenglykolen bzw. deren Monoether zur Verfügung zustellen erfindungsgemäß gelöst werden kann durch Verwendung eines aus Lithiumchlorid und Calciumhydroxid oder Calciumhydroxid allein bestehenden Katalysatorensystems. Die Umsetzungsprodukte aus diesen Verfahren zeichnen sich u. a. dadurch aus, daß sofern Verfärbung auftritt, diese praktisch nicht ins Gewicht fällt. Hervorzuheben ist auch die gute Filtrierbarkeit der Ansätze.

Die vorliegende Erfindung betrifft insbesondere ein Umesterungsverfahren zur Herstellung der (Meth)acrylsäureester der Formel I 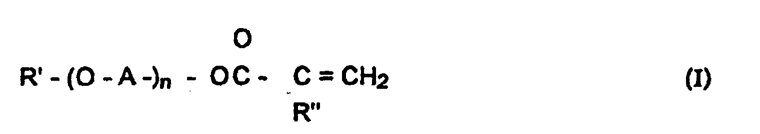 worin
- A: für einen Rest ausgewählt aus der Gruppe bestehend aus
-CH₂ CH₂-, -CH₂ CH₂-CH₂-, 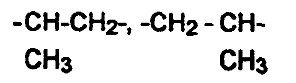
- R': für einen gegebenenfalls verzweigten, gegebenenfalls mit einem Arylrest oder einem Aryloxyrest, substitierten Alkylrest mit 1 bis 28 Kohlenstoffatomen oder für einen Rest 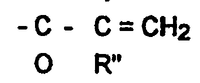
- R": für Wasserstoff oder Methyl und
- n: für eine ganze Zahl von 2 bis 250 insbesondere von 2 bis 50 steht
wobei man einen (Meth)acrylsäureester der Formel II 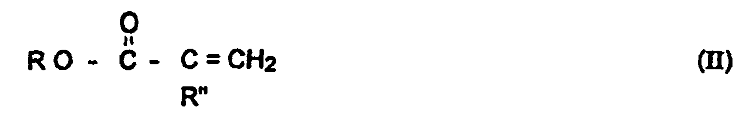 worin
- R: für einen niederen Alkylrest, vorzugsweise C1 - C4-Alkyl steht und R" für die oben bezeichnenden Bedeutungen besitzt
mit dem Alkohol der Formel III

R'''-(O-A-)ₙ-OH (III)

worin
- R''': für Wasserstoff oder für den Rest R' steht und A und n die oben bezeichneten Bedeutungen besitzen in Gegenwart eines Umesterungskatalysators bestehend aus Lithiumchlorid und Calciumhydroxid oder Calciumhydroxid allein umestert.

Unter einem Arylrest bzw. Aryloxyrest als Substituenten in R' sei insbesondere ein Phenyl- bzw. Phenoxyrest verstanden.
Der Umesterungskatalysator bestehend aus Lithiumchlorid und ggf. Calciumhydroxid wird zweckmäßig in katalytischen Mengen, im allgemeinen 0,01 bis 10 Gew.-%, vorzugsweise 0,2 bis 5 Gew.-% bezogen auf die eingesetzten Alkohole der Formel III angewendet. Dabei macht der Anteil des Lithiumchlorids an gesamten Katalysator 0 bis 50 Gew.-%, vorzugsweise 0 bis 30 Gew.-% aus. Als Richtwert seien beispielsweise eine Katalysatorzusammensetzung aus 20 Gew.-% Lithiumchlorid und 80 Gew.-% Calciumhydroxid genannt bei einer Anwendungsmenge von 0,25 Gew.-% bezogen auf das Gesamtgewicht des Reaktionsansatzes.

Zweckmäßigerweise befindet sich der (Meth)acrylsäureester der Formel II im Überschuß über die stöchiometrisch berechnete Menge, jeweils abhängig davon, ob die Alkohole der Formel III ein Diol oder dessen Monoether darstellt. Im allgemeinen wird ein ca. 1,5 bis 10-facher Überschuß über die berechnete Menge - als Richtwert sei ein ca. 5-facher Überschuß am Ester der Formel III über den Alkohol der Formel II genannt - eingesetzt. Bevorzugt wird die Verwendung von Methylmethacrylat oder Methyl- oder Ethylacrylat. Die Alkohole der Formel III umfassen z. B. das Tetraethylenglykol so wie Polyethylenglykole im Molekulargewichtsbereich 380 bis ca. 9000.

Im allgemeinen erübrigt sich die Mitverwendung eines Lösungsmittels. Gegebenenfalls können jedoch inerte (nicht-radikalbildende) Lösungsmittel wie z. B. Kohlenwasserstoffe wie Toluol, Cyclohexan, Hexan, Heptan u. ä. verwendet werden.

Dringend empfiehlt sich die Anwendung mindestens eines stabilisierenden Agens vom Typ der Radikalfänger um die Polymerisation der im Raktionsansatz anwesenden polymerisationsfähigen Verbindungen hin anzuhalten.

Genannt seien Vertreter der Gruppe der Hydrochinonverbindungen, der sterisch gehinderten Phenole, und der sterisch gehinderten Amine sowie der Hydroxylaminderivaten (vgl. Ullmann's Encyclopedia of Industrial Chemistry. Vol. A 20, 460 - 505 VCH 1992).

Als vorteilhaft hat sich z. B. die Anwesenheit von Vertretern von mindestens zwei Gruppen, z. B. der Hydrochinonverbindungen wie z. B. dem Hydrochinonmonomethylether, der sterisch gehinderten Phenole wie z. B. dem 4-Methyl 2,6-di-tert-butylphenol, 2,4-Dimethyl-6-tert-butylphenol, der sterisch gehinderten Amine wie dem 4-Hydroxy-2,2,6,6-tetramethylpiperidinooxyl, sowie der Hydroxylaminverbindungen wie dem N,N-Diethylhydroxylamin erwiesen. Im allgemeinen werden diese stabilisierenden Agentien im ppm-Bereich zugesetzt, beispielsweise im Bereich 50 - 5000 ppm. bezogen auf die Gesamtheit der anwesenden Komponenten.

Als besonders günstig hat sich nach beendeter Alkoholyse der Zusatz einer Verbindung vom Typ der Kieselsäure erwiesen, möglicherweise aufgrund eines gewissen Basenbindungsvermögens. Die Mengen liegen vorzugsweise im Bereich des 1 - 2fachen der eingesetzten Katalysatormenge.

Die Durchführung der Reaktion geschieht zweckmäßig oberhalb Raumtemperatur, vorzugsweise im Bereich 60 - 120°C. Wird das besonders bevorzugte Methylmethacrylat oder -acrylat eingesetzt, so kann zweckmäßig das bei der Umesterung gebildete Methanol am azeotropen Gemisch mit dem Methacrylsäureester (bei 65 - 75°C) abgezogen werden.
Die Reaktionsdauer liegt im allgemeinen bei 1 - 20, vorzugsweise 3 - 5 Stunden.

Die Reaktion kann wie folgt durchgeführt werden:
Der mehrwertige Alkohol, bzw. dessen Monoether der Formel III werden mit dem Überschuß des (Meth)acrylsäureesters und dem Stabilisator in einem geeigneten Reaktionsgefäß vorgelegt Der Katalysator kann während der Reaktion zugesetzt werden, oder er ist von Anfang an anwesend. Bei Zugabe zum Reaktionsgemisch empfiehlt sich die Zugabe in feinverteilter Form im Gemisch, beispielsweise als Pulver oder Granulat.

Die Umsetzung kann beispielsweise wie folgt vorgenommen werden:
Man gibt in einem geeigneten Reaktor mit Rührvorrichtung beispielsweise einen 20 I VA-Kessel mit Doppelankerrührung mit kontrollierter Lufteinleitung und Dampfbeheizung, ausgestattet mit Destillationskolonne, Kolonnenkopf (Flüssigphasenteiler) Kühler, Vorlage und das Gemisch aus dem Alkohol der Formel III, dem niederen (Meth)acrylsäureester der Formel II im Überschuß, den Stabilisatoren, dem Katalysator gebildet aus Lithiumchlorid plus Calciumhydroxid oder Calciumhydroxid allein.

Die Umesterungsreaktion findet vorzugsweise innerhalb einer Gesamtreaktionszeit von ca. 4 - 5 Stunden und bei einer Sumpftemperatur im Bereich > 100°C und < 120°C beispielsweise < 105°C insbesondere bei 110 - 118°C statt.

Nach beendeter Alkoholyse wird bei ca. 80°C die Kieselsäure everbindung zugefügt und über kürzere Zeit, z. B. 10 Minuten lang gerührt.
Die Rohesterlösung wird zweckmäßig der Filtration unterworfen, beispielsweise mittels eines 2 I-Seitz-Druckfilters. Der Filtrationsvorgang erweist sich regelmäßig als völlig problemlos. Dann wird vorteilhaft im Dünnschichtverdampfer eingeengt und kann anschließend mit Wasser auf eine gewünschte Konzentration verdünnt werden.

Man erhält die Substanzen in der Regel eine leicht gelbliche, viskose Flüssigkeit, die bei Raumtemperatur zu einer weißen, wachsartigen Masse erstarrt. Mit Wasser gemischt wird beispielsweise - eventuell nach einer weiteren Filtration ein nahezu farbloser, klarer Ester der Formel I erhalten.

Den erfindungsgemäß erhältlichen (Meth)acrylsäureester der Formel I kommt als (Co)monomere ein weitgespanntes Spektrum von Anwendungsmöglichkeiten zu. Genannt seien z. B. ihre dispergierenden Eigenschaften bei Abwesenheit basischer stickstoffhaltiger Gruppen.

Die folgenden Beispiele erläutern das erfindungsgemäße Verfahren:

### Beispiel 1

### Herstellung von Methoxypolyethylenglycol-750-methacrylat

In einen 20 I-Edelstahlkessel mit Doppelankerrührer, Lufteinleitrohr, 1 m NW50-Glaskolonne gefüllt mit Packungen der Fa. Sulzer, Typ EX und automatischem Kolonnenkopf (Flüssigphasenteiler), Kühler und Vorlage gibt man 9 kg (12 mol) Methoxypolyethylenglycol-750, 6 kg (60 mol) Methylmethacrylat, 7,5 g Lithiumchlorid und 30 g Calciumhydroxid. Als Inhibitoren werden 0,81 g Hydrochinonmonomethylether, 6,1 g 2,6-Ditert.butyl-4-methylphenol und 1 g N,N-Diethylhydroxylamin zugesetzt.

Unter Lufteinleitung erhitzt man das Reaktionsgemisch auf 110 - 118°C und zieht dabei über den Kolonnenkopf ein Methylmethacrylat-Methanolazeotrop ab, bis nach 4,5 h die Kopftemperatur 99°C erreicht und die Alkoholyse beendet ist. Man kühlt auf 80°C und gibt 45 g Tonsil L80 FF (Fa. Südchemie AG) zu. Nach 10 min. Rühren wird das Reaktionsgemisch druckfiltriert und im Dünnschichtverdampfer DS 50 (Fa. NGW = Normschliff Gerätebau Wertheim) bei 95°C und 12 mbar vom überschüssigen Methylmethacrylat befreit Man erhält 9,7 kg Methoxypolyethylenglycol-750-methacrylat als gelblich-viskose Flüssigkeit, die bei Raumtemperatur zu einer wachsartigen Masse erstarrt. Das Produkt besitzt in 50%ig wäßriger Lösung eine Farbzahl nach APHA von 20.

### Beispiel 2

### Methacrylsäureester eines Fettalkoholethoxylats

Durchführung wie in Beispiel 1, jedoch unter Verwendung von 9,5 kg (7 mol) Lutensol®AT25 (BASF, Umsetzungsprodukt eines C₁₆-C₁₈-Fettalkoholgemischs mit 25 mol Ethylenoxid), 7 kg (70 mol) Methylmethacrylat, 6,5 g Lithiumchlorid und 25,9 g Calciumhydroxid. Als Inhibitoren werden 1,75 g Hydrochinonmonomethylether und 0,175 g Diethylhydroxylamin zugesetzt.

Die Alkoholyse wird in 5,75 h bei Sumpftemperaturen von 109 - 114°C durchgeführt. Nach Reaktionsende kühlt man auf 45°C ab und versetzt den Ansatz mit 64,7 g Tonsil L80 FF (Fa. Südchemie). Nach 10 min. Rühren wird das Reaktionsgemisch mit 5 kg MMA versetzt und unter Zusatz von 130 g Kieselgur Seitz-Super (Fa. Seitz) bei gleicher Temperatur druckfiltriert. Das Filtrat wird mit 19 kg Methylmethacrylat versetzt Man erhält 30 kg einer 25 %igen Lösung des Methacrylsäureesters von Lutensol® AT 25 mit einer Farbzahl nach APHA von 14 und einer Hydroxylzahl < 0,1.

### Beispiel 3

### Herstellung von Methoxypolyethylenglycol-750-methacrylat

In einen 2 I-Rundkolben mit mechanischer Rührung, Lufteinleitrohr, 50 cm-Kolonne NS 29 mit Laborpackungen der Fa. Sulzer Typ EX und automatischem Kolonnenkopf (Flüssigphasenteiler), Kühler und Vorlage gibt man 555 g (0,75 mol) Methoxypolyethylenglycol-750, 370 g (3,7 mol) Methylmethacrylat und 4,9 g Calciumhydroxid. Als Inhibitoren werden 0,063 g Hydrochinonmonomethylether, 0,063 g 2,6-Ditert.butyl-4-methylphenol und 0,126 g Diethylhydroxylamin zugesetzt. Unter Lufteinleitung erhitzt man das Reaktionsgemisch auf 113 - 122°C und zieht dabei über den Kolonnenkopf ein Methylmethacrylat-Methanolazeotrop ab, bis nach 3,75 h die Kopftemperatur 99°C erreicht und die Alkoholyse beendet ist. Die erhaltene Rohesterlösung wird im Rotationsverdampfer eingeengt (75°C Wasserbad, 100 ... 3 mbar Druck) und anschließend durch Zugabe von 605 g Wasser auf eine 50 %ige Lösung des Methoxypolyethylenglycol-750-methacrylats eingestellt. Danach wird die Lösung druckfiltriert. Man erhält 575 g einer 50 %igen Lösung des Produkts in Wasser mit einer Farbzahl von 45 (nach APHA).

## Patentansprüche

1. Verfahren zur Herstellung von (Meth)acrylsäureestern der Formel I 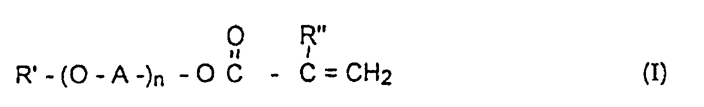 worin
A für einen Rest ausgewählt aus der Gruppe bestehend aus
-CH₂ CH₂-, -CH₂ CH₂-CH₂-, 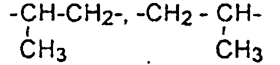
R' für einen geradkettigen oder verzweigten, gegebenenfalls mit einem Arylrest oder einem Aryloxyrest substituierten Alkylrest mit 1 bis 28 Kohlenstoffatomen oder für einen Rest 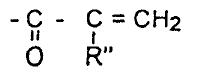
R" für Wasserstoff oder Methyl und
n für eine ganze Zahl von 2 bis 250 steht
in einer Umesterungsreaktion
**dadurch gekennzeichnet**
**daß** man einen (Meth)acrylsäureester der Formel II 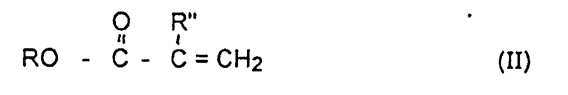 worin
R für einen niederen Alkylrest steht und R" die oben bezeichneten Bedeutungen besitzt
in Gegenwart eines Umesterungskatalysators bestehend aus Calciumhydroxid in Kombination mit Lithiumchlorid mit einem Alkohol der Formel III
R'''- (O - A-)ₙ - OH (III)
worin
R'" für Wasserstoff oder für den Rest R' steht und A und n die oben bezeichneten Bedeutungen besitzen
umestert.

2. Verfahren gemäß Anspruch 1, **dadurch gekennzeichnet, daß** R in Formel II für Methyl, Ethyl oder Butyl steht.

3. Verfahren gemäß den Ansprüchen 1 und 2, **dadurch gekennzeichnet, daß** die Umesterung in Gegenwart mindestens eines an sich bekannten Stabilisators durchgeführt wird.

4. Verfahren gemäß Anspruch 3, **dadurch gekennzeichnet, daß** der Stabilisator ausgewählt wird aus der Gruppe bestehend aus Hydrochinonverbindungen, sterisch gehinderten Phenolen, sterisch gehinderten Aminen und Hydroxylaminderivaten.

5. Verfahren gemäß den Ansprüchen 3 und 4, **dadurch gekennzeichnet, daß** die stabilisierenden Agentien in Mengen von 50 - 5000 ppm. eingesetzt werden.

6. Verfahren gemäß den Ansprüchen 1 - 5, **dadurch gekennzeichnet, daß** die (Meth)acrylsäureestern der Formel II in einem 1,5 bis 10fachen Überschuß über die Alkohole der Formel III eingesetzt werden.

7. Verfahren gemäß den Ansprüchen 1 - 6, **dadurch gekennzeichnet, daß** der Umesterungskatalysator bestehend aus Calciumhydroxid und gegebenenfalls Lithiumchlorid in Mengen von 0,01 bis 10 Gew.-% bezogen auf den Alkohol der Formel III angewendet wird.

8. Verfahren gemäß den Ansprüchen 1 - 7, **dadurch gekennzeichnet, daß** das Gewichtsverhältnis von Lithiumchlorid zu Calciumhydroxid im Bereich 0 zu 100 bis 50 zu 50 liegt.

9. Verfahren gemäß den Ansprüchen 1 bis 8, **dadurch gekennzeichnet, daß** die Umesterung unter Erwärmen durchgeführt wird.

10. Verfahren gemäß Anspruch 8, **dadurch gekennzeichnet, daß** die Umesterung mindestens teilweise im Temperaturbereich > 100°C bis < 120°C durchgeführt wird.

11. Verfahren gemäß den Ansprüchen 1 bis 10, **dadurch gekennzeichnet, daß** das Reaktionsgemisch nach Abschluß der Umesterung einer Filtration unterworfen wird.

## Claims

1. Process for preparing (meth)acrylic acid esters of formula I 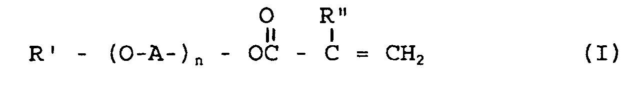 where
A denotes a group selected from among
-CH₂ -CH_{2-, -}CH₂ CH₂-CH_{2-,} 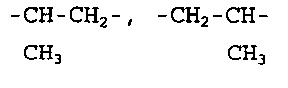
R' denotes a straight-chain or branched alkyl group with 1 to 28 carbon atoms optionally substituted by an aryl group or an aryloxy group, or a group 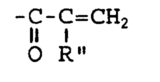
R" denotes hydrogen or methyl and
n denotes an integer from 2 to 250,
in a reaction of transesterification,
**characterised in that** a (meth)acrylic acid ester of formula II 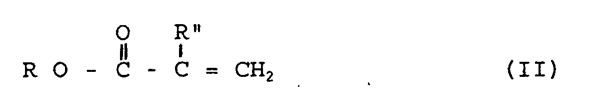 where R denotes a lower alkyl group and
R" has the meanings given above,
is transesterified with an alcohol of formula III
R" ' - (O - A -)ₙ - OH
wherein R"' denotes hydrogen or the group R' and A and n are as hereinbefore defined, in the presence of a transesterification catalyst consisting of calcium hydroxide combined with lithium chloride.

2. Process according to claim 1, **characterised in that** R in formula II denotes methyl, ethyl or butyl.

3. Process according to claims 1 and 2, **characterised in that** the transesterification is carried out in the presence of at least one stabiliser known *per se*.

4. Process according to claim 3, **characterised in that** the stabiliser is selected from among the hydroquinone compounds, sterically hindered phenols, sterically hindered amines and hydroxylamine derivatives.

5. Process according to claims 3 and 4, **characterised in that** the stabilising agents are used in amounts from 50 to 5000 ppm.

6. Process according to claims 1 to 5, **characterised in that** the (meth)acrylic acid esters of formula II are used in a 1.5- to 10-fold excess over the alcohols of formula III.

7. Process according to claims 1 to 6, **characterised in that** the transesterification catalyst consisting of calcium hydroxide and optionally lithium chloride is used in amounts of 0.01 to 10 wt.%, based on the alcohol of formula III.

8. Process according to claims 1 to 7, **characterised in that** the weight ratio of lithium chloride to calcium hydroxide is in the range from 0 : 100 to 50 : 50.

9. Process according to claims 1 to 8, **characterised in that** the transesterification is carried out with heating.

10. Process according to claim 8, **characterised in that** the transesterification is carried out at least partly in a temperature range from >100°C to <120°C.

11. Process according to claims 1 to 10, **characterised in that** after the transesterification has ended the reaction mixture is filtered.

## Revendications

1. Procédé de production d'esters d'acide (méth)acrylique de formule I : 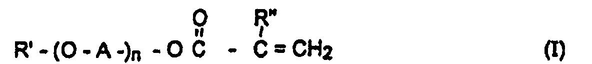 dans laquelle
A représente un reste choisi dans le groupe qui consiste en :
**-CH**_{**2**} **CH**_{**2**}**-, -CH**_{**2**} **CH**_{**2**}**-CH**_{**2**}**-,** 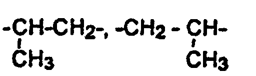
R' représente un reste allyle linéaire ou ramifié éventuellement substitué par un reste aryle ou un reste aryloxy, ayant de 1 à 28 atomes de carbone, ou un reste 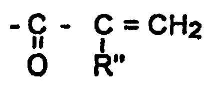
R" représente de l'hydrogène ou un méthyle, n représente un nombre entier de 2 à 250, dans une réaction de transestérification,
**caractérisé en ce qu'**
on transestérifie un ester d'acide (méth)acrylique de formule II 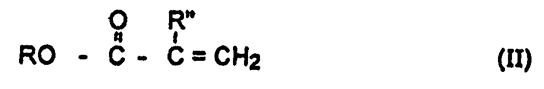 dans laquelle R représente un reste alkyle inférieur et R" possède les significations désignées ci-dessus,
en présence d'un catalyseur de transestérification consistant en de l'hydroxyde de calcium en combinaison avec du dichlorure de lithium, avec un alcool de formule III :
R''' - (O-A)ₙ - OH III
dans laquelle R''' représente de l'hydrogène ou le reste R' et A et n possèdent les significations désignées ci-dessus.

2. Procédé selon la revendication 1,
**caractérisé en ce que**
R dans la formule II représente méthyle, éthyle ou butyle.

3. Procédé conformément aux revendications 1 et 2,
**caractérisé en ce que**
la réaction est effectuée en présence d'au moins un agent stabilisateur connu en soi.

4. Procédé conformément à la revendication 3,
**caractérisé en ce que**
l'agent stabilisant est choisi dans le groupe qui consiste en les composés hydroquinone, les phénols stériquement empêchés, les amines stériquement empêchées, et les dérivés de l'hydroxylamine.

5. Procédé conformément aux revendications 3 et 4,
**caractérisé en ce que**
les agents stabilisants sont mis en oeuvre en quantités de 50-5000 ppm.

6. Procédé conformément aux revendications 1 à 5,
**caractérisé en ce que**
les esters d'acide (méth)acrylique de formule II sont mis en oeuvre en un excès de 1,5 à 10 fois par rapport aux alcools de formule III.

7. Procédé conformément aux revendications 1 à 6,
**caractérisé en ce que**
le catalyseur de transestérification consistant en de l'hydroxyde de calcium et le cas échéant en du chlorure de lithium est utilisé en quantités de 0,01 à 10 % en poids rapporté à l'alcool de formule III.

8. Procédé conformément aux revendications 1 à 7,
**caractérisé en ce que**
le rapport en poids du chlorure de lithium à l'hydroxyde de calcium se situe dans la zone de 0 à 100 à 50 pour 50.

9. Procédé conformément aux revendications 1 à 8,
**caractérisé en ce que**
la transestérification est exécutée tout en chauffant.

10. Procédé conformément à la revendication 8,
**caractérisé en ce que**
la transestérification est exécutée au moins partiellement dans une zone de température allant de > 100°C à < 120°C.

11. Procédé conformément aux revendications 1 à 10,
**caractérisé en ce que**
le mélange réactionnel est soumis après achèvement de la transestérification, à une filtration.
